# EUROPEAN PATENT APPLICATION

(11) **EP 1 787 581 A1**
(43) Date of publication of application: **23.05.2007**
(21) Application number: 05025014.1
(22) Date of filing: 16.11.2005
(51) Int. Cl.: A61B 5/103, A61B 6/00

(54) **Measuring the femoral antetorsion angle y of a human femur in particular on the basis of fluoroscopic images**

(71) Applicant: BrainLAB AG, 85551 Heimstetten (DE)
(72) Inventor: Maier, Christian, 80802 München (DE)
(74) Representative: Schwabe - Sandmair - Marx

(57) **Abstract**

The present invention is directed to a method for defining an antetorsion angle γ of a human femur, said antetorsion angle γ being defined by two angles α and β which can be determined by means of two images of the human femur, by taking the two images such that a co-ordinate system can be assembled, such that an equation can be used to calculate the antetorsion angle γ, wherein said equation reads as follows: tan γ = tan α / (cos β · cos γ).

## Description

The present invention relates to a method for determining an antetorsion angle γ of a human femur, according to claim 1. Furthermore, the invention relates to a data carrier or data storage device according to claim 11 and to a program according to claim 12.

In the prior art, there is a need to determine or measure the antetorsion angle of the human femur. Known methods for measuring the antetorsion angle of the human femur use different means, such as for instance pre-operative X-ray images, CT data or intraoperative fluoroscopic images. Prior art references referring to such methods include: Egund, N., Palmer, J. "Femoral anatomy described in cylindrical coordinates using computed tomography", Acta Radiol. Diagn 1984; 25:209-215; Herman, KL, Egund, N.: "Measuring anteversion in the femoral neck from routine radiographs", Acta Radiol; 1998; 39:410-415; Herman, K.L., Egund, N: "CT measurement of anteversion in the femoral neck: the influence of femur positioning", Acta Radiol 1997; 38:527-532; Murphy, S., Simon, S., Kijewski, P., et al. "Femoral anteversion". Journal of Bone Joint Surgen 1987; 69A:1169-1176; Sugano, N., Noble, P., Kamaric E. "A comparison of alternative methods of measuring femoral anteversion", Journal Computer Assisted Tomography 1998; 22(4):610-614; Hofstetter, R., Slomoczykowski, M., Krettek, C., et al.: "Computer-assisted fluoroscopy-based reduction of femoral fractures and antetorsion correction", Computer Aided Surgery 2000; 5(5):311-325.

Another approach in the prior art relates to a measurement device which has to be inserted into the intramedullary canal of a human femur and thus involves major surgery. This measurement device is described in US Patent No. 5,728,128.

In accordance with the prior art, it is necessary to seriously impact the body of a patient, either by means of high X-ray doses, a surgical operation and/or the like.

Accordingly, it is an object of the present invention to provide a method for measuring or determining an antetorsion angle γ of a human femur, wherein said method is simple and does not impact on the human being or animal.

It is another object of the present invention to provide a method which allows to use small X-ray doses to be used but is nonetheless accurate and provides reliable data for accurately determining the angle in question.

At least one of the above objects is at least partially solved by the method set forth in claim 1. Advantageous embodiments of the method according to the invention are defined in the sub-claims.

The advantages according to the invention are achieved by a method for determining an antetorsion angle γ of a human femur, said antetorsion angle γ being defined by an angle between the central axis through the femoral neck of said human femur, said central axis running through the centre of the femoral head and being parallel to the cortical surface of the proximal part of the femoral neck, and the posterior condyle axis, said posterior condyle axis connecting the most posterior points of the femoral epicondyles of the human femur if the central axis through the femoral neck and the posterior condyle axis are projected onto a plane normal to the femoral shaft axis, wherein the femoral shaft axis is the central axis of the approximately cylindrically shaped shaft of the human femur, the central axis of the cylindrical shaft being parallel to the most anterior tangent to the distal femur, and the femoral shaft axis also being perpendicular to the posterior condyle axis, said method comprising the following steps: taking a first image of the posterior condyles of the human femur, such that the posterior arcs of the condyles overlap laterally, such that the posterior axis can essentially be projected onto a point, and the normal to the image plane is parallel to the posterior condyle axis; defining the essentially most anterior tangent to the anterior cortex of the distal femur; shifting said tangent parallel to itself until it includes the point of the posterior condyle axis, thus determining the transcondylar plane; determining a parallel to the femoral shaft axis. After the first image has been taken and its intended orientation with respect to the transcondylar plane of the human femur has been reached, it is possible that the second image cannot be taken in an optimal relationship to the transcondylar plane. This means that it is possible that there is a tilt angle of the second image with respect to the normal of the transcondylar plane. This tilt angle θ can be determined on the basis of data with respect to an orientation of the camera used to take the second image. In an optimised case, this tilt angle θ should be zero or close to zero. However, if the tilt angle θ occurs and is not negligible, an attempt should be made to avoid needing more than two images, in order to avoid an additional burden on the patient. Therefore, the tilt angle θ needs to be determined, after which the femoral neck axis enclosing the angle α with the transcondylar plane is defined, and the antetorsion angle γ is calculated by means of the formula tan γ = tan α' / (cos β cos θ).

If is possible to avoid the second image being tilted by the angle θ, it is possible to take the second image of the femoral neck of the human femur from a lateral direction, such that the normal to the image plane of the second image encloses an angle β with the femoral shaft axis, but no angle θ.

As set forth above, the angle θ needs to be determined in the following equation: tan γ = tan α /cos β · cos θ. If there is no tilt of the second image with respect to the normal of the transcondylar plane, i.e. cos θ=1, the equation reads as follows: tan γ =tan α /cos β. The latter equation can in particular be used if the second image is taken from said truly lateral direction, as explained in the present patent application.

The central axis through the femoral neck runs through the centre of the femoral head and always has the same distance from the cortical surface of the proximal part of the femoral neck, the central axis should be parallel to said cortical surface.

In accordance with the method according to the invention, a truly lateral image of the posterior condyles is also achieved, wherein the truly lateral image is given when both of the condyles overlap in the lateral fluoroscopic image acquired as the first image. In a truly lateral image, a posterior condyle axis is projected onto a single point and the normal to the image plane is parallel to the posterior condyle axis. This single point on the fluoroscopic image, i.e. in the plane of the fluoroscopic image, defines the posterior condyle axis in the three-dimensional space in which the human femur is to be studied and localised. By means of the same lateral image - i.e. the first image in terms of the method of the invention - of the posterior condyles, the most anterior tangent to the anterior cortex of the distal femur is defined. Since the fluoroscopic image is two-dimensional, the tangent line corresponds to the transcondylar plane, which is acquired by shifting the tangent line parallel to the posterior direction until it includes the posterior condyle axis.

The transcondylar plane achieved in this way is intersected by a plane normal to the posterior condyle axis in order determine the parallel to the femoral shaft axis. In the direction of the femoral shaft axis is the normal of the projection plane onto which the femoral neck axis and the posterior condyle axis are projected. The second or axial image of the femoral neck then has to be taken. The axial image is a common projection which is preferably used by trauma surgeons. As can be seen in the method according to the invention, other directions may also be used, wherein the second image has to be taken from a lateral direction. A navigation system such as Vector Vision/Trauma 2.5, both products of the applicant, can be used to indicate whether the imaging direction is parallel to the transcondylar plane. It is of course preferable to use the navigation system such that the correct imaging direction can be indicated before the second or axial image is taken. The normal to the image plane of the axial image encloses the angle β with the femoral shaft axis, the orientation of which corresponds in a preferred case to the Y-axis in the three-dimensional co-ordinate system, which is also preferred in accordance with the present invention. The surgeon can define the femoral neck axis on the basis of the second image. The femoral neck axis encloses the angle α with the transcondylar plane. Lastly, by means of the angles α and β, it is possible to calculate the antetorsion angle γ using the above-mentioned equation tan γ = tan α / cos β.

The method of the present invention is advantageous in that the antetorsion angle can be acquired by direct measurement, to a high accuracy. It is only minimally necessary to take two fluoroscopic images, such that the radiation exposure for the patient can be dramatically reduced. In addition, it is not necessary to perform invasive surgery into the body of a patient. On the other hand, accuracy can be further improved by fixing a reference array or marker array to the femoral bone. However, it is also possible to fix the reference or marker array to an operation table on which the patient and thus the femur of the patient is located.

According to one advantageous embodiment of the method according to the invention, at least one of the first and second images is a fluoroscopic image. At least one of the steps performed before the second image is taken should be performed on the basis of the first image.

Furthermore, it is preferable to acquire the parallel by intersecting the transcondylar plane with a plane normal to the posterior condyle axis as already indicated above.

According to one advantageous embodiment, the second image to be taken is an axial image of the femoral neck, in order to correspond to the usual procedure employed by a surgeon.

The second image is preferably taken from a lateral direction, wherein before imaging, a computer-aided navigation system determines whether an imaging direction is parallel to the transcondylar plane.

According to a further improved variant of the method according to the invention, the femoral neck axis can be determined by means of the second image. Furthermore, at least one of the first and second images is acquired by using a stereotactic computer-aided surgery system. In particular, the computer-aided surgery system should be able to use the images and information calculated and/or inputted by an operator to transform the corresponding data into a three-dimensional co-ordinate system.

According to another preferred embodiment, it is advantageous to fix a tracking device or marker array to the human femur or to a base on which the human femur is placed.

According to another aspect of the present invention, a data carrier or storage device is provided which comprises a program for realising the method according to the present invention or according to at least one of the advantageous variants of the method according to the present invention.

According to a further aspect of the invention, a program is provided which, when executed on a computer or computer system realises the steps according to the method of the invention or realises one of the advantageous embodiments of the method according to the invention.

The invention comprises a procedure for measuring the antetorsion angle of the human femur. The antetorsion angle is defined as the angle between the femoral neck axis and the posterior condyle axis when projected onto a plane normal to the femoral shaft axis. The femoral neck axis is defined as the central axis through the femoral neck. It runs through the centre of the femoral head and is parallel to the cortical surface of the proximal part of the femoral neck. The posterior condyle axis connects the most posterior points of the femoral epicondyles. The femoral shaft axis is the central axis of the cylindrical shaft axis and is perpendicular to the posterior condyle axis. From these axes, it is possible to define a co-ordinate system in which the antetorsion angle can easily be measured by definition.

The transcondylar plane which includes the posterior condyle axis and is parallel to the femoral shaft axis is defined. The femoral shaft axis is defined by intersecting the plane tangential to the anterior cortex with a plane perpendicular to the posterior condyle axis. Once the femoral neck axis has been defined, it is possible to calculate the angle α between the femoral neck axis and the transcondylar plane. This angle α is then projected onto a plane perpendicular to the femoral shaft axis, in order to obtain the antetorsion angle γ. The posterior condyle axis and the femoral neck axis are each defined on a single fluoroscopic image, resulting in a total of two images for determining the antetorsion angle.

The present invention is directed to a method for defining an antetorsion angle γ of a human femur, said antetorsion angle γ being defined by two angles α and β which can be determined by means of two images of the human femur, by taking the two images such that a co-ordinate system can be assembled, such that an equation can be used to calculate the antetorsion angle γ, wherein said equation reads as follows: tan γ = tan α / cos β. If the second image is additionally tilted by an angle θ with respect to the normal of the transcondylar plane, then tan α = tan α / cos θ.

The invention will now be described with reference to the attached drawings and referring to one preferred embodiment according to the method of the present invention,
wherein the drawings show:
- Fig. 1: a human femur showing the orientation of the parts of the human femur relative to a co-ordinate system;
- Fig. 1a: the elements of the antetorsion angle γ;
- Fig. 1b: another embodiment of the elements of the antetorsion angle γ;
- Fig. 2: a lateral image of the distal femur; and
- Fig. 3: an axial image of the femoral neck in combination with a corresponding perspective view of the remainder of the human femur.

In the figures, the same elements are designated by the same reference numbers, in order to avoid referring to the same element.

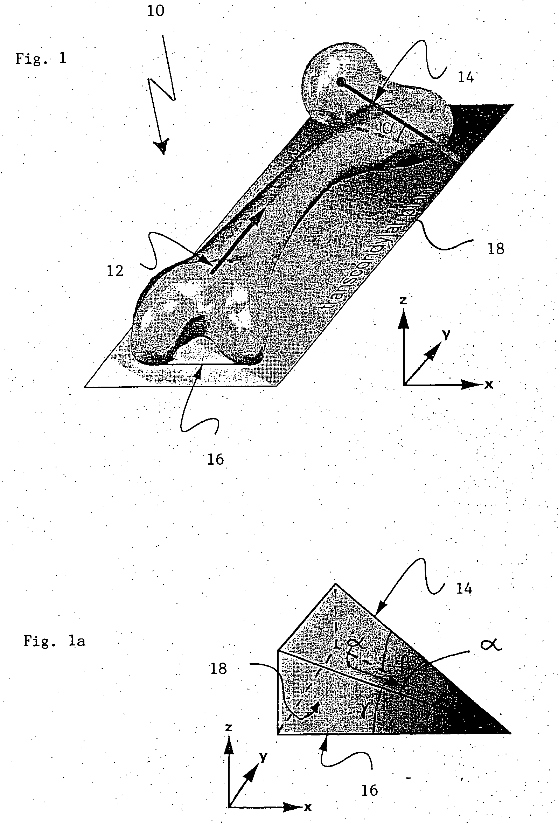

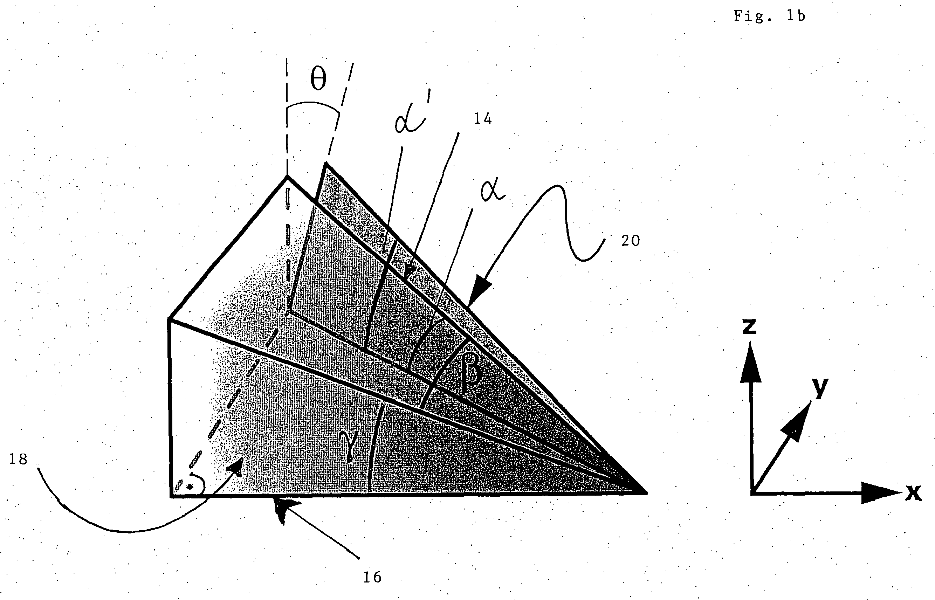

Fig. 1 shows a human femur 10. The human femur has a particular orientation relative to a XYZ coordinate system. A transcondylar plane 18 is shown and is arranged parallel to the plane of the X and Y axes. A posterior condyle axis 16 is defined, as will be described in the following and this posterior condyle axis 16 is also a line within the transcondylar plane 18. A tangent 12 to the anterior cortex of the human femur 10 is parallel to the transcondylar plane 18. A femur neck axis 14 of the neck of the human femur 10 is elevated from the transcondylar plane 18, and the projection of the femoral neck axis 14 onto the transcondylar plane 18 encloses an angle α.

The important angles α, β and γ are again shown in Fig. 1a, but in a more abstract form with respect only to the principal directions of the posterior condyle axis 16, the femoral neck axis 14 and the transcondylar plane 18 and with respect to the XYZ co-ordinate system, but not with respect to the particular human femur represented by the geometrical elements 14, 16, 18.

Fig. 1b shows another embodiment according to the invention. The difference with respect to Fig. 1a is that the second image of the femoral neck of the human femur is not taken from a lateral direction, but is tilted with respect to the normal of the transcondylar plane. Accordingly, a plane 20 of the second image is tilted by an angle θ with respect to the normal of the transcondylar plane 18. This angle θ can be measured, for example by measuring a tilt angle of the camera taking the second image. The tilt angle θ can be ensured relative to the orientation of the camera when taking the first image and thus with respect to the transcondylar plane 18. Due to this tilt angle θ, the femoral neck axis encloses an angle α' with the transcondylar plane 18 which is different to the angle α according to Fig. 1b. If θ is close or equal to zero, α' is approximately or identically equal to α. If θ occurs, the equation for calculating γ reads as follows: tan γ'/(cos β cos θ).

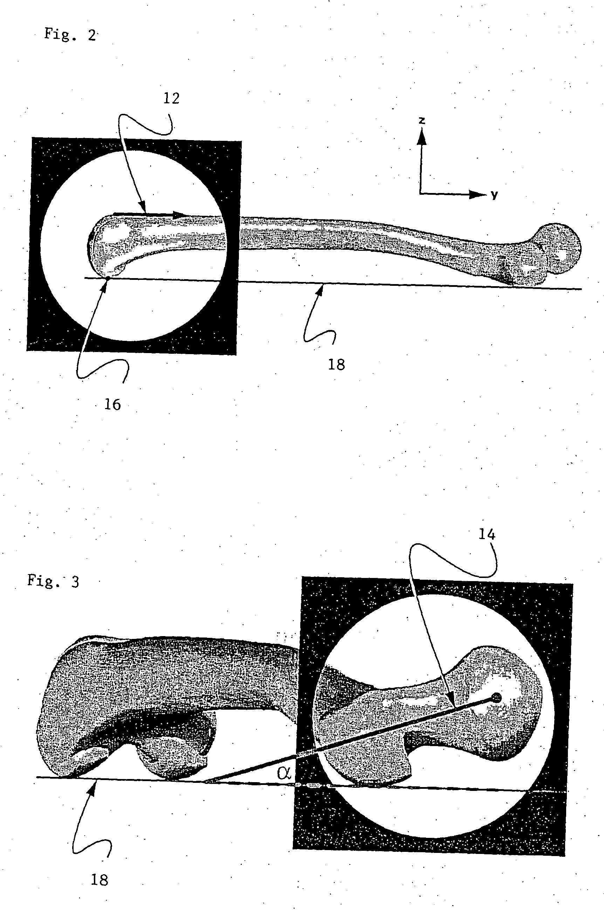

Fig. 2 shows a lateral image of the distal femur. In this truly lateral image, the posterior condyles overlap. Therefore, the posterior condyle axis is a single point to be identified by the surgeon or by a particular software analysing the corresponding truly lateral fluoroscopic image, e.g. the second image according to the invention. In addition, the surgeon can be responsible for defining a most anterior tangent 12 to the anterior cortex of the distal femur in the second image, although this can also be accomplished by an image-analysing computer program.

In summary, Fig. 2 yields the transcondylar plane 18 which should then be used afterwards for taking the second image in a truly lateral orientation.

In Fig. 3, the femoral neck has been recorded to provide a special axial image, wherein the normal of the image is parallel to the transcondylar plane 18. The image direction points from a truly lateral position towards the human femur and in particular towards the femoral neck. The surgeon can also be responsible for defining the femoral neck axis 14 in this image although this task can also be realised by a suitable image analysing software. The femoral neck axis 14 encloses an angle α with the transcondylar plane 18.

In accordance with a preferred embodiment of the present invention, the method according to the invention can be achieved by firstly taking a lateral image or truly lateral image of the posterior condyles. Such a truly lateral image is shown in Fig. 2, in which the posterior condyle axis appears to be a single point 16, which means that the two posterior condyles overlap in the first fluoroscopic image according to the invention, i.e. a lateral fluoroscopic image. The term "truly" in connection with the lateral image, i.e. the first image according to the invention, means that the posterior condyle axis is projected onto the single point, as stated above, and the normal to the image plane in parallel to the posterior condyle axis 16. By identifying of this single point in the fluoroscopic image, the surgeon can spatially the posterior condyle axis. This first image of the posterior condyles can be used to define the most anterior tangent 12 to the anterior cortex of the distal femur. The two-dimensional nature of the first image and the orientation of the first image perpendicular to the second image is helpful in using a first fluoroscopic image as a plane in the XYZ co-ordinate system, i.e. in three-dimensional space. The plane of the first image can be shifted parallel to the posterior direction until it includes the posterior condyle axis 16, in order to define the transcondylar plane 18.

The transcondylar plane 18 can then be intersected with a plane normal to the posterior condyle axis 16, to provide a parallel to the femur shaft axis which is parallel to the Y axis of the XYZ co-ordinate system shown in the figures. Said parallel is the normal of the projection plane onto which the femoral neck axis 14 and the posterior condyle axis 16 are projected. The second image then has to be taken of the femoral neck, in the orientation indicated in Fig. 3. The second image is an axial image and is a common projection which is often used by trauma surgeons. This image is necessarily taken from a lateral direction, such that the two images can be assembled to provide a three-dimensional system, i.e. an orientation in the XYZ co-ordinate system. It is preferable to acquire the second image by using a navigation system to help identify whether the imaging direction is parallel to the transcondylar plane 18. The normal to the image plane of the second image encloses the angle β with the femoral shaft axis which is parallel to the Y axis of the XYZ co-ordinate system mentioned in the figures.

By means of the second image, the operator or surgeon can define the femoral neck axis which encloses the angle α with the transcondylar plane 18. It is then possible to use the co-ordinate system defined by the planes of the two images to calculate the antetorsion angle γ by means of the equation tan γ = tan α / cos β. If, the orientation of the second image is tilted with respect to the normal of the transcondylar plane 18 (tan α = tan α' / cos θ), the tilt angle θ has to be taken into account.

## Claims

1. A method for determining an antetorsion angle of a human femur (10), said antetorsion angle γ being defined by an angle between
- the central axis through the femoral neck of said human femur (10), said central axis running through the centre of the femoral neck and being parallel to the cortical surface of the proximal part of the femoral neck, and
- the posterior condyle axis (16), said posterior condyle axis connecting the most posterior points of the femoral condyles of the human femur (10) if the central axis through the femoral neck and the posterior condyle axis are projected onto a plane normal to the femoral shaft axis, wherein the femoral shaft axis is the central axis of the approximately cylindrically shaped shaft of the human femur, the central axis of the cylindrical shaft being parallel to the most anterior tangent (12) to the distal femur, and the femoral shaft axis also being perpendicular to the posterior condyle axis (16); said method comprising the following steps:
(a) taking a first image of the posterior condyle of the human femur (10), such that the two posterior condyles overlap laterally, such that the posterior condyle axis (16) can essentially be projected onto a point, and the normal to the image plane is parallel to the posterior condyle axis (16);
(b) defining the essentially most anterior tangent (12) to the anterior cortex of the distal femur;
(c) shifting said tangent parallel to itself until it includes the point of the posterior condyle axis (16), thus determining the transcondylar plane (18);
(d) determining a parallel to the femoral shaft axis;
(e) taking a second image of the femoral neck of the human femur such that the normal to the image plane of the second image encloses an angle β with the femoral shaft axis and an angle θ with the transcondylar plane (18);
(f) defining the femoral neck axis enclosing the angle α' with the transcondylar plane (18); and
(g) calculating the antetorsion angle γ by means of the equation tan γ = tan α / (cos θ).

2. The method of claim 1, wherein the second image is taken from a lateral direction, such that cos θ = 1 or approaches 1, and the antetorsion angle γ is calculated by means of the modified equation tan γ = tan α/cos β.

3. The method according to any one of claims 1 or 2, wherein at least one of the first and second images is a fluoroscopic image.

4. The method according to any one of claims 1 or 3, wherein at least one of steps (a) to (d) is performed on the basis of the first image.

5. The method according to any one of claims 1 to 4, wherein the parallel according to step (d) is acquired by intersecting the transcondylar plane (18) with a plane normal to the posterior condyle axis (16).

6. The method according to any one of claims 1 to 5, wherein the second image to be taken is an axial image of the femoral neck.

7. The method according to any one of claims 2 to 5, wherein the second image is taken from a lateral direction, wherein before imaging a computer-aided navigation system determines whether an imaging direction is parallel to the transcondylar plane (18).

8. The method according to any one of claims 1 to 7, wherein the femoral neck axis according to step (f) is defined by means of the second image.

9. The method according to any one of claims 1 to 8, wherein at least one of the first and second images is acquired using a stereotactic computer-aided surgery system.

10. The method according to any one of claims 1 to 9, wherein the information calculated and/or inputted by an operator on the basis of the first and second images can be is transformed in order to project the corresponding data into a three-dimensional co-ordinate system.

11. The method according to any one of claims 1 to 10, wherein a tracking device is fixed to a human femur or a base, e.g. an operation table, on which the human femur is placed.

12. A data carrier or storage device which comprises a program for realising the method according to at least one of claims 1 to 11.

13. A program which, when executed on a computer or computer system, realises the steps according to at least one of claims 1 to 11.
